# EUROPÄISCHE PATENTSCHRIFT

(11) **EP 1 127 034 B1**
(45) Veröffentlichungstag und Bekanntmachung des Hinweises auf die Patenterteilung: **14.05.2003**
(21) Anmeldenummer: 99971790.3
(22) Anmeldetag: 04.11.1999
(51) Int. Cl.: C05F 17/02, B01F 13/02

(54) **VERFAHREN UND VORRICHTUNG ZUR AUFBEREITUNG EINES ORGANIK ENTHALTENDEN STOFFGEMISCHES**
METHOD AND DEVICE FOR PREPARING A MIXTURE OF SUBSTANCES CONTAINING ORGANIC COMPONENTS
PROCEDE ET DISPOSITIF DE PREPARATION D'UN MELANGE DE MATERIAUX CONTENANT DES COMPOSANTS ORGANIQUES

(30) Priorität: 06.11.1998 DE 19851306; 03.03.1999 DE 19909353
(43) Veröffentlichungstag der Anmeldung: 29.08.2001
(73) Patentinhaber: Müller, Patrick, 4127 Birsfelden (CH)
(72) Erfinder: Müller Patrick, CH-4102 Binningen (CH); Widmer Christian, CH-4102 Binningen (CH)
(74) Vertreter: Polte, Willi, Dr.-Ing. Dipl.-Ing.
(86) Internationale Anmeldenummer: IB9901950
(87) Internationale Veröffentlichungsnummer: WO00027777

(56) Entgegenhaltungen:
- WO-A-95/20554
- WO-A-97/27158
- DE-A- 4 301 116
- DE-A- 19 608 586
- DE-A- 19 648 731
- US-A- 5 066 392

## Beschreibung

Die Erfindung betrifft ein Verfahren zur Aufbereitung eines Strukturanteile und Organik enthaltenden Stoffgemisches gemäß dem Oberbegriff des Patentanspruchs 1 und eine Vorrichtung insbesonders zur Durchführung dieses Verfahrens.

Ein derartiges Verfahren wird beispielsweise zur Aufbereitung von Restabfall eingesetzt. In der DE 196 48 731 A1 ist ein Abfallaufbereitungsverfahren beschrieben, bei dem der Restabfall in einem Perkolator aufbereitet wird. Durch eine derartige Perkolation oder Extraktion werden durch ein Extraktions- oder Waschmittel organische Bestandteile, anorganische Substanzen sowie ggf. wasserlösliche Fettsäuren aus dem Abfall ausgewaschen. Der Rückstand wird aus dem Perkolator entnommen und nach einer sich anschließenden Trocknung einer Verbrennung zugeführt oder deponiert.

Es zeigte sich, daß sich mit diesem Verfahren die organischen Substanzen nicht in dem erforderlichen Umfang aus dem Restabfall entfernen lassen.

Die Nachteile dieses bekannten Abfallaufbereitungsverfahrens lassen sich durch das in der WO 97/27158 A1 offenbarte Verfahren zur Behandlung von biologischen Abfällen ausräumen. Bei diesem Verfahren wird ein neuartiger Perkolator eingesetzt, bei dem der Abfall den Reaktor in Horizontalrichtung (Längsrichtung) durchläuft und dem Perkolationsprozeß eine biogene Reaktion durch Zuführung von Luftsauerstoff (Prozessluft) überlagert wird.

Durch die Zufuhr von Prozessluft werden die Zellen der Organik aufgebrochen und die freigesetzten organischen Substanzen durch die Auswaschflüssigkeit abtransportiert. Zur Vermeidung einer Kanalbildung innerhalb des Abfalls und zum Einbringen von Scherkräften ist im Reaktor ein Rühr- oder Umwälzwerk vorgesehen, durch das der Abfall in Vertikalrichtung (parallel zur Durchströmungsrichtung der Auswaschflüssigkeit und der Prozessluft) durchmischt und auch in Transportrichtung bewegt wird.

Nachteilig bei diesem Verfahren ist, daß zur Führung und Durchmischung des Abfalls innerhalb des Reaktors ein erheblicher Aufwand betrieben werden muß, der die Investitionskosten wesentlich beeinflußt. Ein derartig komplexer mechanischer Aufbau birgt auch die Gefahr eines Anlagenausfalls aufgrund einer Störung im Transportsystem des Reaktors in sich, so daß ein vergleichsweise hoher Aufwand zur Wartung des Reaktors betrieben werden muß. Derartige Ausfälle des Reaktors aufgrund der erforderlichen Wartung oder einer Störung in der Reaktorperipherie können jedoch nur durch Bereitstellen von entsprechenden Pufferräumen aufgefangen werden, in die der Abfall während der Stillstandszeit des Reaktor zwischengelagert werden kann. In der DE 196 08 586 A1 ist ein Rotteverfahren beschrieben, bei dem die Miete mit Druckluft beaufschlagt ist.

Demgegenüber liegt der Erfindung die Aufgabe zugrunde, ein Verfahren zur Aufbereitung eines Strukturanteile und Organik enthaltenden Stoff gemisches sowie eine Vorrichtung zu schaffen, bei denen bei minimalem vorrichtungstechnischen Aufwand ein hinreichender Abbau des organischen Anteils erfolgt.

Diese Aufgabe wird hinsichtlich des Verfahrens durch die Merkmale des Patentanspruchs 1 und hinsichtlich der Vorrichtung durch die Merkmale des Patentanspruchs 11 gelöst.

Durch die Maßnahme, das Strukturanteile und Organik enthaltende Stoffgemisch (beispielsweise Restabfälle) den Reaktor ohne wesentliche Längs- und Quervermischung durchlaufen zu lassen und die Känalbildung durch das Beaufschlagen des Stoffgemisches mit etwa parallel oder quer zur Bewegungsrichtung gerichteten Kräften zu verhindern, kann der Reaktor wesentlich einfacher als beim vorbeschriebenen Stand der Technik aufgebracht werden, da kein Rührwerk zur Quervermischung vorgesehen werden muß. Die Kräfte werden vorzugsweise vom Randbereich des Reaktors, durch Druckluft eingeleitet. Bei der Verwendung von Druckluft wird das Haufwerk auch mit Scherkräften beaufschlagt, über die die Oberfläche der Schüttung umgebildet und Stoffgemischpartikel aufgefasert werden.

Der Reaktor kann dabei als Perkolator und als Trockner eingesetzt werden, ohne daß es irgendwelcher Umbauten oder Umrüstungen bedarf.

Beim Einbringen der die Kanalbildung verhindernden Kräfte über die Austragseinrichtung wird das Stoffgemisch vorzugsweise zumindest teilweise im Umlauf geführt, so daß durch die den Umlauf bewirkenden Förderelemente Scherkräfte eingeleitet werden.

Die erfindungsgemäße Strömungsführung erlaubt es, den Reaktor sehr kompakt auszubilden, wobei sämtliche Zufuhrund Abfuhreinrichtungen am Kopf- bzw. Bodenteil des Reaktors angeordnet sein können.

Die Kräfte zur Veränderung einer Kanalbildung und die zur Oberflächenneubildung und zum Aufreissen der Par-tikel erforderlichen Scherkräfte werden über Druckluft aufgebracht, die vom Randbereich des Reaktors her in das Haufwerk (Schüttung) eingeblasen wird. Durch die Druckluft wird die Schüttung partial expandiert, so daß eine Oberflächenneubildung im Haufwerk erfolgt und die Partikel aufgrund der eingetragenen Scherkräfte aufgerissen werden - die Stoffaustauschfläche für den Aufschluß des Stoffgemisches durch den Luftsauerstoff und durch die Auswaschflüssigkeit wird erhöht.

Bei einem besonders bevorzugten Ausführungsbeispiel werden die Druckluft und die Prozessluft durch Düsen zugeführt, die im Fußteil und/oder im Bodenteil des Reaktors angeordnet sind.

Erfindungsgemäß wird es bevorzugt, wenn das Stoffgemisch den Reaktor im wesentlichen vertikal (parallel zur Schwerkraftrichtung) oder horizontal durchläuft, so daß das Stoffgemisch etwa parallel oder in Querstrom zur Prozessluft geführt wird.

In dem Fall, in dem der Reaktor als Perkolator verwendet wird, wird die Auswaschflüssigkeit vorzugsweise über einen Verteiler im Kopfteil des Reaktors zugeführt.

Die Druckluft wird mit einem Druck von mehr als 2 bar, vorzugsweise mehr als 4 bar zugeführt, während die Prozessluft üblicherweise mit einem Druck von 0,5 bar beaufschlagt ist.

Die Düsen zum Einbringen der Prozess- und/oder Druckluft sind vorteilhafter Weise individuell ansteuerbar, so daß ein bestimmtes Druckluftprovil über den Reaktorquerschnitt einstellbar ist.

Die Verwendung von Druckluft zum Einbringen von Scherkräften und zur Verhinderung der Kanalbildung hat den Vorteil, daß gleichzeitig der für den aerob ablaufenden Prozeß im Haufwerk erforderliche Luftsauerstoff zugeführt wird, so daß die Druckluft praktisch eine Doppelfunktion erfüllt:
1. Zufuhr von Luftsauerstoff für den aeroben Abbau und
2. Eintragen von Scherkräften.

Bei einem einfacheren nicht erfindungswesentlichen Ausführungsbeispiel werden die Kräfte zur Verhinderung einer Kanalbildung im Haufwerk beispielsweise durch eine Austrageinrichtung zugeführt, die am Bodenteil des Reaktors angeordnet ist. Diese Austrageinrichtung kann beispielsweise eine Kratzbodenanlage oder eine ähnliche, das Stoffgemisch schichtweise abführende Fördereinrichtung sein. Diese Variante hat den weiteren Vorteil, daß durch die Vorschubbewegung der Austrageinrichtung Zuführ- und Abführöffnungen im Bodenteil des Reaktors freigehalten werden, so daß die Auswaschflüssigkeit austreten und Druck- bzw. Prozessluft in das Haufwerk eintreten kann. Als Austragseinrichtung kann auch ein Förderschneckenteppich, ein Walking-Floor, Silofräsereinrichtungen etc. verwendet werden. Diese Austragseinrichtungen können selbstverständlich auch bei dem vorstehend beschriebenen erfindungsgemäße Ausführungsbeispiel mit Druckluft verwendet werden.

Da das Stoffgemisch den Reaktor vorzugsweise schichtförmig durchläuft, kann die Verweilzeit des Stoffgemisches innerhalb des Reaktors bei einer kontinuierlichen Prozeßführung sehr genau bestimmt werden, so daß die Durchlaufzeiten hinsichtlich des biologischen Abbaus optimierbar sind. Bei den eingangs genannten Lösungen konnte aufgrund der Längs- und Quervermischung mittels des Rührwerkes lediglich ein mittlerer Verweilzeitwert festgelegt werden.

Die beladene Prozessluft bzw. die beladene Druckluft werden einer Abgasreinigung zugeführt, bei der organische Bestandteile abgetrennt und die gereinigte Luft in den Prozeß zurückgeführt wird.

Die Energiebilanz der Anlage läßt sich weiter verbessern, wenn das beladene Auswaschmittel einer Abwasserreinigung zugeführt wird. Diese kann eine Biogasanlage enthalten, in der die Umsetzung der Organik in Biogas erfolgt. Durch Energiekopplung des freigesetzten Biogases läßt sich der erfindungsgemäß Prozeß weitestgehend energieautark gestalten.

Bei der vorgeschriebenen Verfahrensführung wird das Organik enthaltende Stoffgemisch einer sogenannten Hydrolyse unterzogen, bei der durch Zusammenwirken der Luft und der Auswaschflüssigkeit das organische Material durch die aerobe, thermophile Erwärmung durch die Luft gelöst und angesäuert und durch die Auswaschflüssigkeit abtransportiert wird. D.h. der Abbau der organischen Bestandteile erfolgt durch Einstellung einer gewissen Feuchtigkeit und durch Zuführung von Reinluft.

Die Weiterverarbeitung des Stoffgemisches sieht erfindungsgemäß eine Trocknung des Rückstandes vor. Diese Trocknung kann mit minimalem energetischen Aufwand durch eine aerobe, thermophile Erwärmung des Rückstandes im Reaktor erfolgen. Dazu kann das Stoffgemisch entweder im Reaktor mit Reinluft beaufschlagt werden, so daß durch die resultierende aerobe Erwärmung Wasserdampf über die zugeführte Luft ausgetragen und der Rückstand getrocknet wird. Die Trocknung und Hydrolyse in einem einzigem Reaktor setzt allerdings einen absatzweisen Betrieb voraus, so daß dies nur bei kleineren Anlagen in Frage kommen dürfte. Bei größeren Anlagen wird ein eigener Reaktor (Trockner) zur aeroben, thermophilen Erwärmung des Rückstandes aus der Hydrolyse vorgesehen. Selbstverständlich können diese beiden Reaktoren in einem einzigen Behältnis in n-facher Folge nacheinander geschaltet werden, so daß sich mehrere Hydrolyse-/Trocknungsvorgänge hintereinander anschließen können.

Die Energiebilanz der Anlage läßt sich weiter verbessern, wenn das beladene Auswaschmittel einer Abwasserreinigung zugeführt wird. Diese kann eine Biogasanalge enthalten, in der die Umsetzung der Organik in Biogas erfolgt. Durch Energiekopplung des freigesetzten Biogases läßt sich der erfindungsgemäße Prozeß energieautark gestalten.

Ganz besonders vorteilhaft ist es, wenn die derart aufbereitete Feststoffaktion nach der Hydrolyse und/oder der aeroben Trocknung einer Kompaktierung zugeführt wird. Dabei wird der einen gewissen Partikeldurchmesser aufweisende Feststoff in eine vorbestimmten geometrische Form, beispielsweise Pellets oder Briketts gepreßt. Durch diese Kompaktierung erfolgt eine weitere Entwässerung des aufbereitenden Stoffgemisches, so daß nach der Kompaktierung ein trockenstabiler nicht mehr eluierbarer Körper vorliegt.

Dieser Körper kann beispielsweise als Ersatzbrennstoff als Alternative zu fossilen Energieträgern oder in einer Deponie abgelagert werden.

Die Hauptanwendung des erfindungsgemäßen Verfahrens dürfte bei der Behandlung vom Abfall liegen; prinzipiell läßt sich das Verfahren jedoch auch bei anderen Stoffgemischen anwenden, die organische Bestandteile aufweisen.

Als Waschmittel wird üblicherweise Wasser eingesetzt, das beim erfindungsgemäßen Verfahren im Kreislauf gefahren wird. Die Luft für die Hydrolyse und thermophile Trocknung des Stoffgemisches 'kann im Gegenstrom zum Stoffgemisch oder aber auch im Gleichstrom geführt werden.

Sonstige vorteilhafte Weiterbildungen der Erfindung sind Gegenstand der weiteren Unteransprüche. Im folgenden werden bevorzugte Ausführungsbeispiele der Erfindung anhand schematischer Rechnungen näher erläutert. Es zeigen:
Fig. 1 einen Schnitt durch einen nicht erfindungswesentlichen Reaktor, in dem eine Hydrolyse eines organische Bestandteile enthaltenden Stoffgemisches erfolgt;
Fig. 2 einem nicht erfindungswesentlichen Reaktor zur Durchführung einer aeroben, thermophile Trocknung;
Fig. 3 eine nicht erfindungswesentiche Anlage, bei der mehrere Hydrolyse- und Trocknungsreaktoren gemäß den Figuren 1 und 2 hintereinander geschaltet sind;
Fig. 4 eine nicht erfindungswesentliche Vorrichtung, bei der in einem gemeinsamen Behältnis mehrere Reaktoren zur Hydrolyse und/oder Trocknung hintereinander geschaltet sind;
Fig. 5 eine Draufsicht auf die Vorrichtung aus Figur 4 und die
Fig. 6 und 7 erfindungsgemäße Ausführungsformen eines Reaktors.

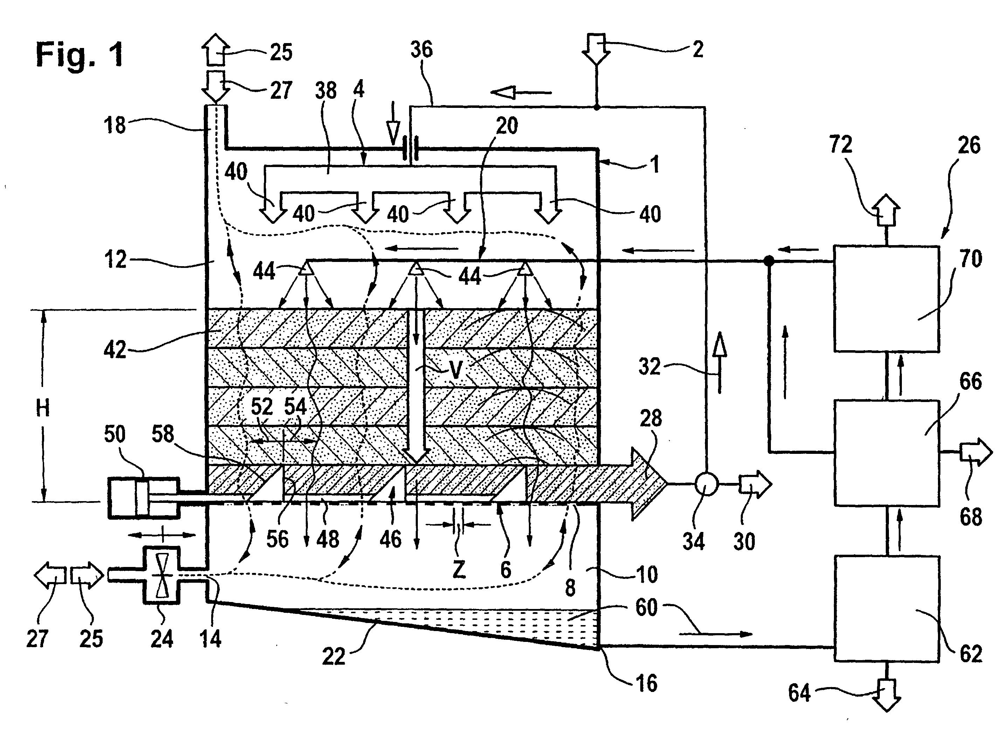

Figur 1 zeigt ein Verfahrensschema zur Erläuterung eines nicht erfindungwesentlichen Verfahrens und der Vorrichtung zur
Durchführung des Verfahrens. Demgemäß erfolgt die aerobe Hydrolyse (aerobe biogene Reaktion und Perkolation) in einem Reaktor 1, dem das aufzubereitende Stoffgemisch 2 über eine Materialeintrageinrichtung 4 zugeführt wird. Das aufzubereitende Stoffgemisch enthält einen hohen Anteil an Strukturmaterial und Organik. Derartige Stoffgemische treten beispielsweise bei Hausmüll, Biomüll, Gewerbemüll etc. auf.

Der Reaktor 1 ist als abgeschlossener Behälter ausgeführt, so daß die im folgenden noch näher beschriebenen Stoffströme über Schleusen-, Ventileinrichtungen etc. zugeführt werden.

Der eigentliche Reaktor 1 ist vorzugsweise ein Stahloder Betonbehälter, der beim gezeigten Ausführungsbeispiel von oben mit dem Stoffgemisch (Restmüll) beschickt wird.

Ein substantieller Anteil der organischen Fraktion des Stoffgemisches besteht aus kurzkettigen Verbindungen, die meist an eine Oberfläche absorbiert sind. Wird diese Oberfläche von warmen Wasser umspült, werden auch primär nichtlösliche Verbindungen hydrolysiert und ausgewaschen. Der Hydrolysegrad hängt von der Aufenthaltszeit im Reaktor 1 ab. Die geruchsintensiven Komponenten des Stoffgemischs und die Hydrolyseprodukte sind gut wasserlöslich und können ausgewaschen werden. Mit der Perkolation erreicht man daher eine Reduktion der Organik und eine Desodorierung des Stoffgemisches. Mit der Auswaschflüssigkeit (Prozeßwasser) werden auch Feinsandpartikel ausgetragen. Der Reaktor 1 ist geruchsdicht verschlossen und die Abluft wird in dem folgenden noch näher beschriebenen Weise desodoriert.

Bei der Perkolation wird zusätzlich auch Prozessluft zugeführt, durch die der physikalisch-chemische Effekt der Wasserextraktion durch Steigerung des bakteriellen Abbaus verstärkt wird. Die Mikroorganismen beginnen in der aeroben Umgebung Exoenzyme auszuscheiden, welche partikuläre polymere Komponenten zu Monomeren spalten und in Lösung bringen.

Während der Perkolation werden ca. 10 % der Inertstoffen (Glas, Keramik, Sand) freigesetzt, welche mit der Auswaschflüssigkeit ausgetragen werden. Die Abscheidung erfolgt in einem Sandklassierer, welcher gleichzeitig eine Nachspülung zur Sandwäsche erlaubt.

Bei dem Ausführungsbeispiel gemäß Fig. 1 ist die Eintrageinrichtung 4 an dem in Schwerkraftrichtung gesehen oberen Endabschnitt des Reaktors 1 angeordnet.

Im unteren Bereich des Reaktors 1 ist zumindest eine Austrageinrichtung 6 ausgebildet, über die das aufbereitete und biologisch aufgeschlossene Stoffgemisch aus dem Reaktor 1 abführbar ist.

Der Reaktor 1 hat desweiteren unterhalb (Darstellung nach Figur 1) der Austrageinrichtung 6 einen Sammler 10, der von einem Reaktionsraum 12 über einen Siebboden 8 abgetrennt ist. Die im folgenden noch näher beschriebene Austrageinrichtung 6 ist derart ausgebildet, daß das auf dem Siebboden 8 liegende Stoffgemisch schichtförmig aus dem Reaktor abgeführt und die Öffnungen des Siebbodens 8 durchgängig gehalten werden.

Im Sammler 10 münden ein Luftanschluß 14 und ein Auswaschflüssigkeits-Austritt 16. Im Kopfbereich des Reaktors 1 sind ein weiterer Luftanschluß 18 und ein Auswaschmittel-Verteiler 20 angeordnet.

Die zur Perkolation oder Extraktion der organischen Bestandteile des Stoffgemisches verwendete Auswaschflüssigkeit (Wasser) wird über den Verteiler 20 in den Reaktor eingespeist und über den Austritt 16 abgezogen. Zur Vereinfachung der Strömungsführung fällt der Boden 22 des Reaktors 1 zum Austritt 16 hin ab, so daß sich die Auswaschflüssigkeit im Bereich des Austritts 16 sammelt.

Der in Figur 1 untere Luftanschluß 14 ist mit einer Luftfördereinrichtung 24 verbunden. Je nach Bauart der Luftfördereinrichtung (Gebläse, Verdichter) läßt sich innerhalb des Reaktors 1 eine Strömung 25 vom unteren Luftanschluß 14 zum oberen Luftanschluß 18 oder eine Strömung 27 in umgekehrter Richtung vom oberen Luftanschluß 18 zum unteren Luftanschluß 14 einstellen. D.h., entsprechend der Bauart der Luftfördereinrichtung 24 wird das im Reaktor 1 aufgenommene Stoffgemisch in der Darstellung gemäß Figur 1 von unten nach oben oder von oben nach unten mit Luft durchströmt.

Die Auswaschflüssigkeitsströmung erfolgt in Schwerkraftrichtung, d.h. von dem im Reaktor 1 oben angeordneten Verteiler 20 zum Austritt 16.

Die aus dem Reaktor 1 austretende Auswaschflüssigkeit wird über eine im folgenden noch näher beschriebene Abwasseraufbereitungseinrichtung 26 (Anaerobfilter) aufbereitet und dann im Kreislauf zurück zum Verteiler 20 geführt.

Der auf dem Siebboden 8 aufliegende Rückstand wird als Materialaustrag 28 über die Austrageinrichtung 6 abgezogen und entweder als Produkt 30 einer weiteren Verarbeitung zugeführt oder aber als Umlaufgut 32 zur Eintrageinrichtung 4 zurückgeführt. Die Aufteilung des Materialaustrags 28 in Produkt 30 und/oder Umlaufgut 32 erfolgt über einen geeigneten Dosierer 34, der beispielsweise als Schieber, Klappe, Weiche etc. ausgeführt sein kann.

D.h. durch geeignete Einstellung des Dosierers 34 kann ein Teil des Materialaustrages 28 als Umlaufgut 32 in den Reaktor 1 zurückgeführt werden, und dort zur Impfung des Stoffgemisches und somit zur Beschleunigung des biologischen Abbaues eingesetzt werden.

Desweiteren werden durch die Umlaufführung des ganzen oder eines Teils des Stoffgemisches über die Fördermittel Scherkräfte ins Umlaufgut eingebracht, so daß Oberflächen des Stoffgemisches neu gebildet und Partikel aufgefasert werden.

Zum besseren Verständnis sei nunmehr die einzelnen vorbeschriebenen Bauelemente der erfindungsgemäßen Vorrichtung detaillierter erläutert.

Das eintretende Stoffgemisch 2 wurde zuvor in bekannter Weise mechanisch aufbereitet, so daß es eine vorbestimmte maximale Partikelgröße aufweist. Dieses aufbereitete Stoffgemisch 2 wird über geeignete Fördereinrichtungen, beispielsweise Förderbänder 36 der Eintrageinrichtung 4 zugeführt, über die eine Verteilung des Stoffgemisches 2 über den Reaktorquerschnitt erfolgt. Beim gezeigten Ausführungsbeispiel hat die Eintrageinrichtung 4 einen Querförderer 8, über den das Stoffgemisch in der Zeichenebene und quer zur Zeichenebene verteilt und über den Querschnitt verteilten Materialabwurftrichtern 40 dem Reaktor 1 zugeführt wird.

Durch Ansteuerung der Materialabwurftrichter 40 oder der Querförderer 38 wird das Stoffgemisch 2 schichtweise in den Reaktor 1 eingebracht, so daß praktisch auf dem Siebboden 8 n-Schichten 42 übereinander liegend angeordnet sind.

Die Füllhöhe H des Reaktors 1 ist so gewählt, daß sich der Verteiler 20 für die Auswaschflüssigkeit oberhalb des Haufwerks befindet. Der Verteiler 20 kann beispielsweise eine Vielzahl von über den Reaktorquerschnitt verteilten Sprühköpfen 44 aufweisen, über die die Auswaschflüssigkeit gleichmäßig über der obersten Schicht 42 verteilbar ist.

Die Austrageinrichtung 6 ist bei dem in Figur 1 dargestellten Ausführungsbeispiel als Horizontalförderer ausgebildet, der derart ausgelegt ist, daß die jeweils untere, auf dem Siebboden 8 aufliegende Stoff gemischschicht in Horizontalrichtung abführbar ist. Bei dem dargestellten Reaktor 1 ist die Austrageinrichtung 6 als Schub- oder Kratzboden ausgeführt, wie er beispielsweise in der WO 95/20554 A1 beschrieben ist. Derartige Schubböden werden beispielsweise in Klärschlammsilos, Kompostierungsanlagen etc. eingesetzt und sind aus dem Stand der Technik bekannt, so daß im folgenden lediglich die wesentlichen Bauelemente beschrieben werden.

Gemäß Figur 1 hat der Schubboden mehrere in Horizontalrichtung (Ansicht nach Figur 1) beabstandete Förderkeile 46, die auf einer Schubstange 48 angeordnet ist. Die Schubstange 48 läßt sich über einen Hydraulikzylinder 50 oder über eine sonstige Antriebseinrichtung parallel zu den Pfeilen 52, 54 in Figur 1 hin und her bewegen.

Die zur Austragöffnung weisende vordere Fläche der Förderkeile 46 ist als Vertikalfläche 56 ausgebildet, während die rückwärtige Fläche eine Schrägfläche 58 ist. Durch entsprechende Ansteuerung des Hydraulikzylinders 50 wird die Schubstange 48 periodisch hin und her bewegt, wobei bei der Bewegung der Schubstange 48 in Pfeilrichtung 52 (nach links in Figur 1) das Stoffgemisch der untersten Schicht entlang der Schrägfläche 58 aufgleitet und in dem Raum hinter dem betreffenden Förderkeil 46 zu liegen kommt. Bei der sich anschließenden Rückbewegung der Schubstange 48 in Pfeilrichtung 54 wird dieses Material durch die Vertikalfläche 56 mitgenommen und nach rechts zum benachbarten Förderkeil 46 oder zur Austragsöffnung gefördert. D.h. die Höhe der Förderkeile 46 bestimmt die Schichthöhe des ausgetragenen Stoffgemisches. Um die Extraktionsbedingungen im Reaktor 1 konstant zu halten, entspricht die Schichtdicke des Materialaustrags etwa der Schichtdicke des Materialeintrags, so daß die Füllhöhe H im wesentlichen konstant bleibt.

Wie bereits eingangs erwähnt, kann ein Teil des Materialaustrags 28 als Impfstoff (Umlaufgut 32) zur Fördereinrichtung 36 oder direkt zur Eintrageinrichtung 4 zurückgeführt werden. Prinzipiell ist auch möglich, den gesamten Materialaustrag 28 als Umlaufgut 32 zu fahren, so daß das Stoffgemisch den Reaktor 1 mehrmals durchläuft und erst nach beispielsweise 4 Durchläufen als Produkt 30 abgeführt wird.

Der unterhalb der Austrageinrichtung 6 angeordnete Siebboden 8 hat eine Maschenweite Z, die in Abhängigkeit von der Zusammensetzung und Partikelgröße des aufzubereitenden Stoffgemisches gewählt ist. Die Konstruktion des schubstange 48 und der Förderkeile 46 ist so gewählt, daß der Siebboden 8 durch die Hin- und Herbewegung des Kratzbodens gereinigt wird, so daß ein Zusetzen der Maschen verhinderbar ist.

Durch den schichtförmigen Materialaustrag wird bewirkt, daß sich das Stoffgemisch in Vertikalrichtung schichtweise von oben nach unten (Figur 1) durch den Reaktor 1 bewegt.

Wie bereits vorstehend erwähnt, kann die Luftfördereinrichtung 24 als Gebläse oder Verdichter ausgebildet werden, so daß sich unterschiedliche Luftströmungsrichtungen im Reaktor 1 einstellen lassen. In beiden Fällen sind die Eintritts- und Austrittsbereiche des Reaktors 1 so gewählt, daß die Luft über dem gesamten Reaktorquerschnitt verteilt das geschichtete Stoffgemisch durchströmt. Diese Luftströmung ist in der Darstellung nach Figur 1 mit gestrichelten Linien angedeutet.

Die Auswaschflüssigkeit durchströmt das geschichtete Stoffgemisch entlang den durchgezogenen Pfeilen von oben nach unten und tritt durch den Siebboden 8 mit Organik beladen in den Sammler 10 ein. Die beladene Auswaschflüssigkeit 60 wird über den Austritt 16 abgezogen und der Abwasseraufbereitungseinrichtung 26 zugeführt. Diese hat einen Störstoffabscheider 62, in dem Störstoffe 64, wie beispielsweise Sand, Steine, Schwimmstoffe, Schwebstoffe etc. abgeschieden werden. Derartige Störstoffabscheider können beispielsweise einen Absetzbehälter und einen Skimmer zur Abscheidung der genannten Störstoffe 64 aufweisen.

Die von den Störstoffen befreite und kolloidale organische Verbindungen in wässriger Phase enthaltende Auswaschflüssigkeit wird dann einem Anaerobfermeter 66, beispielsweise einer Biogas- oder Faulturmanlage zugeführt. In dieser anaeroben Abwasserbehandlung werden als Stoffwechselendprodukte Methan und Kohlendioxid und ggf. in geringen Mengen Schwefelwasserstoff gebildet. Dieses als Abbauprodukt erhaltene Biogas kann in geeigneten BHKW-Anlagen zu Strom und Wärme umgewandelt werden. Ein Teil der aus dem Biogas gewonnenen Energie wird in den erfindungsgemäßen Prozeß zurückgeführt, so daß dieser weitgehend energieautark geführt ist.

Vorversuche zeigten, daß bei der Behandlung von einer Tonne zugeführten Hausmülls ca. 80 Nm³ Biogas mit einem Energieinhalt von 6,5 kWh gewonnen werden können.

Bei dem vorbeschriebenen Ausführungsbeispiel ist dem Reaktor eine Abwasserreinigungsanlage zugeordnet. Alternativ könnte die Auswaschflüssigkeit auch in eine bestehende Kläranlage eingebunden werden oder direkt in die Kanalisation eingeleitet oder einem anderen Behandlungsschritt zugeführt werden. Als Zulauf würde dann Frischoder Betriebswasser oder ein schwach belastetes Abwasser benutzt.

Im Anschluß an den Anaerobfermenter 66 schließt sich eine zweistufige aerobe Nachbehandlung 70 an, wobei Faulwasser aus der Biogasanlage zur Minimierung der Restfracht nachbehandelt und Stickstoff eliminiert wird.

Das dabei entstehende befrachtete Abwasser 72 wird je nach Belastung und geltenden gesetzlichen Vorschriften an einer weiteren Behandlungsstufe zugeführt oder direkt in die Kanalisation eingeleitet. Die in der aeroben Biologie 70 gereinigte Auswaschflüssigkeit wird dann über den Verteiler 20 dem Reaktor 1 zugeführt. Wie in Figur 1 angedeutet ist, kann ein Teilstrom des Faulwassers aus dem anaerob Fermenter 66 unter Umgehung der 2-stufigen aeroben Biologie 70 direkt dem Verteiler zugeführt werden, um katalytisch auf den biologischen Aufschluß im Reaktor 1 zu wirken.

Durch die erfindungsgemäße Strömungsführung innerhalb des Reaktors 1 stellt sich eine aerobe Hydrolyse ein, wobei durch die das Stoffgemisch 2 durchströmende Luft und die über die Auswaschflüssigkeit eingestellte Feuchtigkeit des Stoffgemisches eine aerobe, thermophile Erwärmung stattfindet, durch die die Zellen der Organik aufgebrochen und die freigesetzten organischen Substanzen durch die Auswaschflüssigkeit ausgetragen werden.

Für den Abbau des organischen Materials ist zum einen der aerobe Abbau des verfügbaren Kohlenstoffes C zu CO₂ (Kohlensäure) und zum anderen das Auswaschen der gelösten und angesäuerten Organik und der Abtransport über die Auswaschflüssigkeit verantwortlich. Aufgrund der aeroben, thermophilen Reaktion und des gleichzeitigen Abbaus der organischen Verbindungen steigt die Temperatur im Stoffgemisch während des Extraktionsvorganges (beispielsweise auf ca. 40 bis 50° C) an. Durch diese Temperaturerhöhung wird Wasserdampf freigesetzt, der über die zugeführte Luft ausgetragen wird. Dieser mit der Luft ausgetragene Wasserdampf kann als Kondensat der vorbeschriebenen Abwassserreinigung zugeführt werden.

Die aus dem Reaktor 1 abströmende Luft ist mit Kohlendioxid als Abbauprodukt und dem durch die Erwärmung entstandenen Wasserdampf beladen. Die mit organischen Komponenten beladene Abluft kann einem Biofilter zugeführt werden, in dem eine biologische Reinigung mittels aerober Mikroorganismen erfolgt.

Als Auswaschflüssigkeit wird zunächst reines Wasser verwendet, das nach dem Anfahren der Anlage und dem Erreichen nahezu stationärer Prozeßparameter durch während der aeroben Behandlung aufgelöste Salze in einen sauren Zustand überführt wird. Die leichte Versäuerung des Wassers unterstützt die Auswaschung von löslichen organischen, anorganischen Substanzen und wasserlöslichen Fettsäuren.

Wie in Figur 1 desweiteren dargestellt ist, wird das sich innerhalb des Reaktors 1 befindliche Stoffgemisch 2 durch die Hin- und Herbewegung der Förderkeile 46 mit stoßförmigen Impulsen beaufschlagt, so daß Scher-, Querund Längskräfte in das Stoffgemisch eingebracht werden, durch die etwa auftretende Strömungskanäle der Ausweichflüssigkeit und der Luft zerstört werden. Die Größe dieser Kräfte ist dabei so ausgelegt, daß sie einerseits groß genug sind, um diese Kanäle und Kamine zu zerstören, andererseits jedoch nicht zu einer Zerstörung des Schichtaufbaus führen.

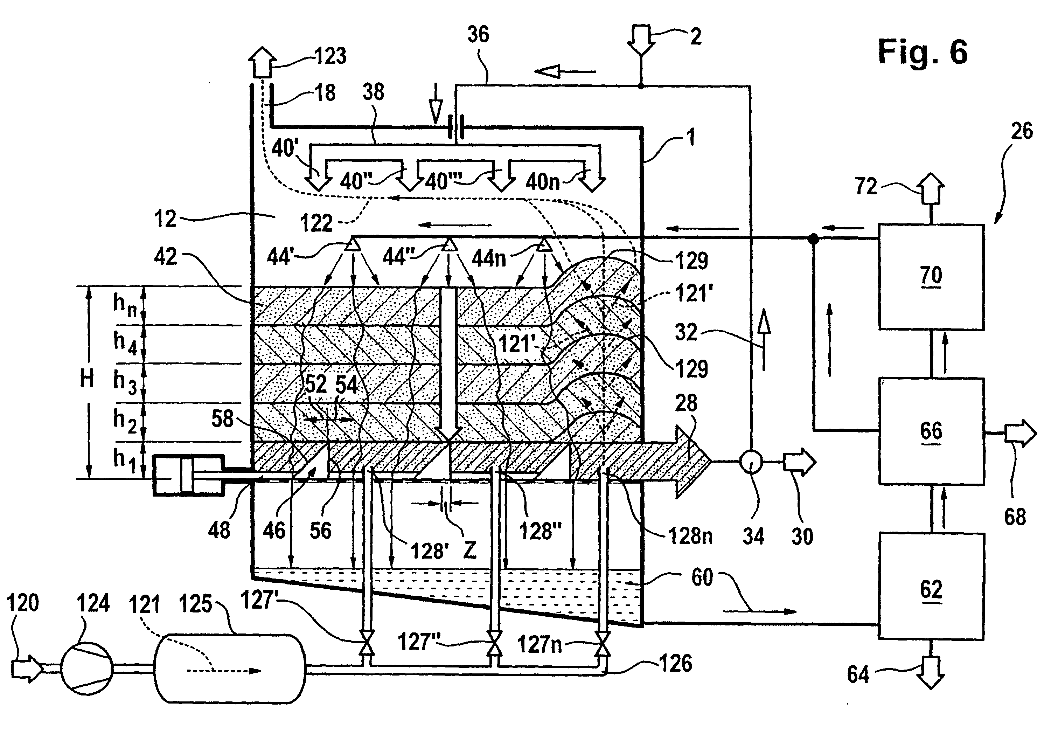

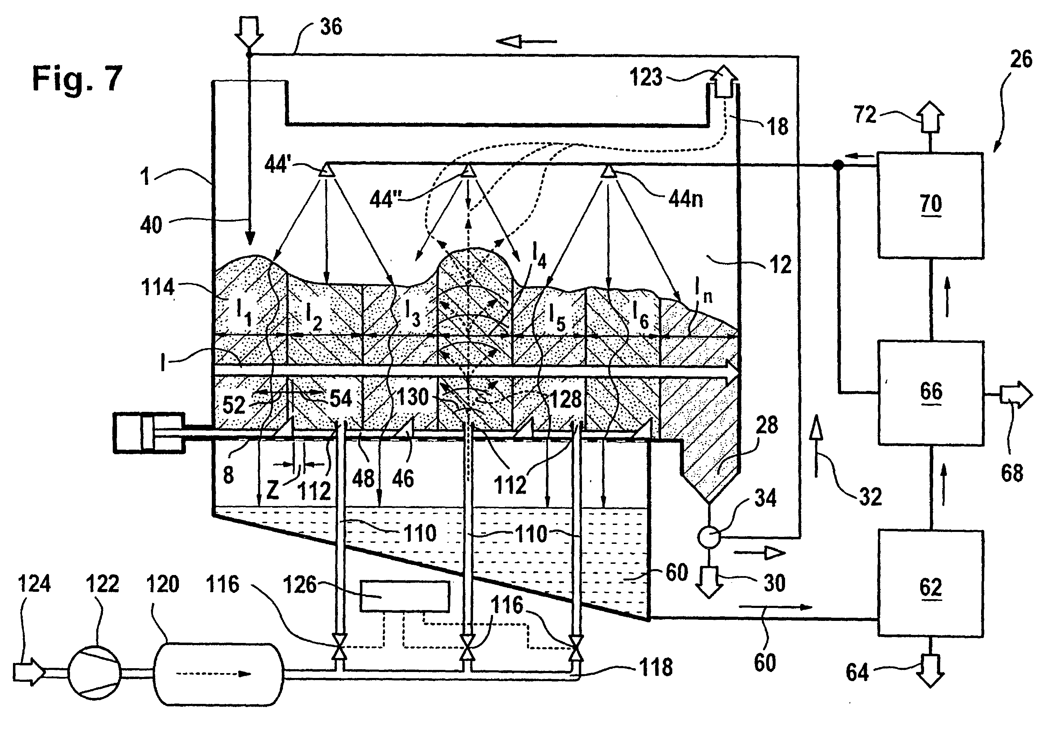

Bei dem in Figur 1 dargestellten nicht erfindungswesentlichen Ausführungsbeispiel werden diese Impulse durch die Bewegung des Kratzbodens herbeigeführt. Erfindungsgemäß werden wie in den Figuren 6 und 7 dargestellt andere Erreger zur Beaufschlagung des Stoffgemisches 2 mit Scherkräften und zur Zerstörung der Kanäle eingesetzt werden wie in den Figuren 6 und 7 dargestellt.

Nach der vorbeschriebenen Hydrolyse, d.h. dem Aufschluß der organischen Bestandteile und der Extraktion dieser Bestandteile mittels der Auswaschflüssigkeit wird der Materialaustrag 28 einer Trocknung zugeführt. Als besonders vorteilhaft hat es sich erwiesen, wenn diese Trocknung als aerobe Trocknung erfolgt, da dann die Restfeuchte mit einem minimalen Energieaufwand verringerbar ist. Eine derartige aerobe Trocknung läßt sich beispielsweise bewirken, in dem die Zufuhr der Auswaschflüssigkeit über den Verteiler 20 unterbrochen wird, so daß das Stoffgemisch 2 nach der Hydrolyse lediglich noch von der Luft durchströmt wird. Durch die Durchströmung des feuchten Stoffgemisches 2 erfolgt ein weiterer aerober Abbau des noch verfügbaren Kohlenstoffes C zu Kohlendioxid. Desweiteren wird, ähnlich wie bei Hydrolyse aufgrund des mikrobiellen Umsatzes das Stoffgemisch erwärmt und dadurch Wasserdampf über die durchströmende Luft ausgetragen. Durch den aeroben Abbau des Kohlenstoffes und die Abführung des Wasserdampfes wird die Restfeuchte des Stoffgemisches reduziert, wobei sich der gewünschte Trockensubstratanteil auf einfache Weise durch die Dauer der aeroben Trocknung einstellen läßt.

Beim vorbeschriebenen Ausführungsbeispiel werden somit die Hydrolyse und die aerobe Trocknung in einem einzigen Reaktor 1 durchgeführt. D.h., der Reaktor 1 läßt sich ohne Modifikation sowohl zum Trocknen als auch zum Perkolieren einsetzen, so daß ein einfacher Anlagenaufbau gewährleistet ist.

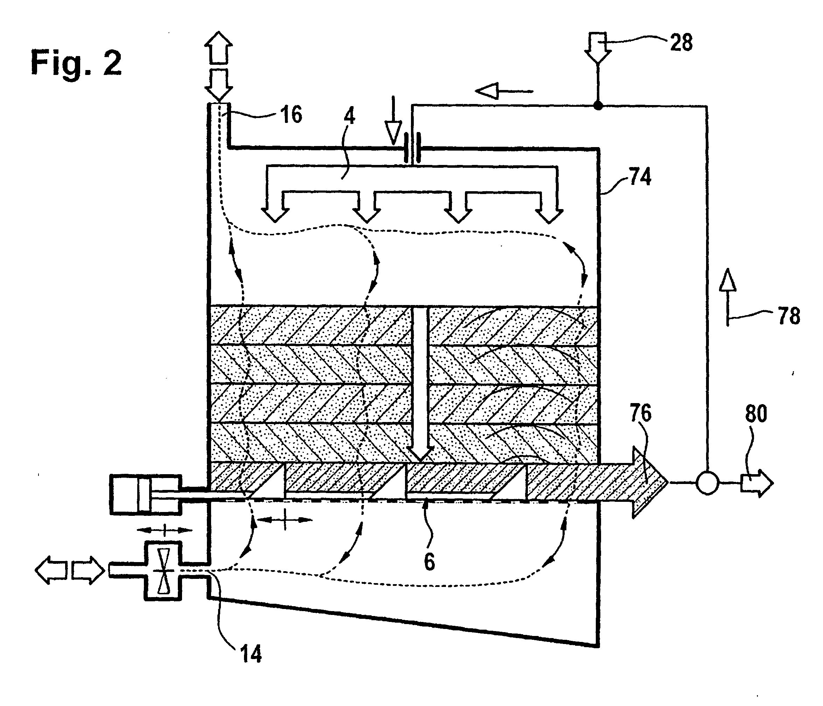

Alternativ dazu könnte dem Reaktor 1 aus Figur 1 ein Trockner gemäß Figur 2 nachgeschaltet werden, dem der Materialaustrag 28 des Reaktors 1 zugeführt wird. Diese aerobe Trockner 74 hat im wesentlichen den gleichen Aufbau wie der Reaktor 1 aus Figur 1, d.h. das Stoffgemisch, in diesem Fall der Materialaustrag 28 wird über eine Eintrageinrichtung 4 in einem mit Schleusen versehenen Behälter eingeführt und nach erfolgter aerober Trocknung über eine Austrageinrichtung 6 abgeführt. Der Trockner 74 hat im Unterschied zum vorbeschriebenen Reaktor 1 mehrere Luftanschlüsse 14 senkrecht zur Zeichenebene übereinanderliegend angeordnet, so daß die Luft flächig eingeblasen werden kann. Die Trocknungsluft kann wiederum im Gegenstrom oder im Gleichstrom zum Stoffgemischstrom geführt werden und wird entsprechend über Luftanschlüsse 14, 16 zu- bzw. abgeleitet.

Im Unterschied zum Reaktor aus Figur 1 hat der Trockner 74 aus Figur 2 keinen Verteiler 20 zum Aufbringen von Auswaschflüssigkeit.

Beim aeroben Trockner 74 ist wiederum eine Teilrückführung des am Ausgang des Trockners 74 anliegenden Trockengutes 76 als Umlaufgut 78 und/oder die Abführung eines getrockneten Produktes 80 vorgesehen. Das zu trocknende Stoffgemisch durchläuft den Trockner 74 vorzugsweise wiederum geschichtet, wobei die Kanalbildung durch impulsförmig aufgebrachte Scher-, Quer- und Längskräfte unterbunden wird. Selbstverständlich könnte dieses 2-stufige Verfahren auch durch zwei hintereinander geschaltete Reaktoren gemäß Figur ersten durchgeführt werden, wobei im ersten Reaktor die Hydrolyse durch Zuführung von Luft und Auswaschflüssigkeit erfolgt, während im zweiten nachgeschalteten Reaktor 1 lediglich die aerobe Trocknung durch Zuführung von Luft erfolgt.

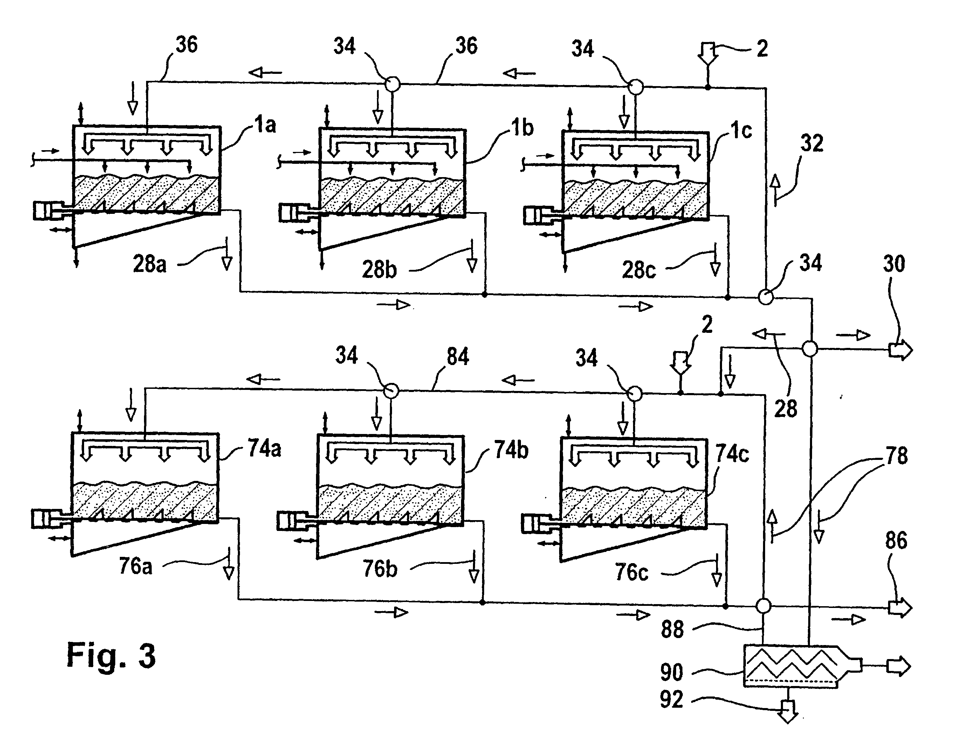

Figur 3 zeigt ein Ausführungsbeispiel, bei dem drei Reaktoren 1a, 1b, 1c gemäß Figur 1 mit drei Trocknern 74a, 74b, 74c gemäß Figur 2 zusammengeschaltet sind. Demgemäß ist den drei Reaktoren 1a, 1b, 1c eine gemeinsame Fördereinrichtung 36 zugeordnet, über die das Stoffgemisch 2 den einzelnen Reaktoren 1a, 1b, 1c zuführbar ist. Über geeignete Dosiereinrichtungen 34 läßt sich wiederum der Stoffstrom zu den einzelnen Reaktoren 1a, 1b, 1c einstellen.

Der Materialaustrag 28a, 28b, 28c aus den einzelnen Reaktoren 1a, 1b, 1c kann wiederum über den Dosierer 34 als Umlaufgut 34 zurückgeführt werden oder aber als Produkt 30 einer weiteren Verarbeitung oder als Materialaustrag 28 der aeroben Trocknung zugeführt werden. Dabei wird der Materialaustrag 28 aus den Reaktoren 1a, 1b, 1c über eine Fördereinrichtung 84 und geeignete Dosierer 34 den Trocknern 74a, 74b, 74c zugeführt.

Im Anlagenschema gem. Figur 3 ist desweiteren vorgesehen, daß das Stoffgemisch 2 auch direkt, d.h. auch unter Umgehung der Reaktoren 1 der Trocknung zugeführt wird. Dies ist beispielsweise dann der Fall, wenn das vorliegende Stoffgemisch bereits einen erheblichen Anteil an Trockensubstanz aufweist, so daß keine Naßwäsche mehr erfolgt.

Der Materialaustrag aus den Trocknern 74, d.h. das Trockengut 76a, 76b, 76c wird dann entweder als Trockenprodukt 86 weiterverarbeitet, als Umlaufgut 78 wieder der Trocknung zugeführt oder als Zwischenprodukt 88 einer Einrichtung 90 zur Entwässerung und/oder Kompaktierung zugeführt.

Die Einrichtung 90 wird auch zur Weiterverarbeitung des Materialaustrages aus den in Figuren 1, 2 dargestellten Reaktoren/Trocknern verwendet. Die Einrichtung 90 kann beispielsweise als Extruder oder Trocknungs-/Strangpresse ausgebildet werden, so daß durch die mechanische Einwirkung und die durch den Druckaufbau entstehende Wärme eine weitere Entwässerung oder Trocknung des Zwischenproduktes 88 erfolgt.

In der Einrichtung 90 wird der extrahierte Restmüll auf einen TS-Gehalt von > 60 % eingestellt. Bei einem bevorzugten Ausführungsbeispiel enthält die Einrichtung 90 desweiteren eine Hochdruckpresse, über die das extrahierte, entwässerte Material pelletierbar ist. Dabei werden Dichten von 1,7 t/m³ erreicht. Der Energieaufwand zur Herstellung der Pellet beträgt ca. 1 % des Energiegehaltes der Pellets, wenn man von einem mittleren Energiegehalt von 14 MJ/Kg ausgeht.

Je nach Ausgestaltung der Einrichtung 90 kann das entwässerte Endprodukt 92 als Pellet, Brikett oder sonstiger kompaktierter Form vorliegen. Durch die vorbeschriebenen Verfahrensschritte läßt sich ein nicht eluierbares und nicht atmungsaktives Produkt herstellen, das sich durch einen hohen Anteil an Trockensubstanz auszeichnet, wobei im Unterschied zu den bekannten Verfahren keine thermische Fremdenergie zur Trocknung aufgewendet werden muß.

Das über die Einrichtung 90 entwässerte Material kann einer Nachtrocknung mittels Kompostierung oder Bandtrocknung unterzogen werden. Üblicherweise wurde bisher nach einer mechanisch-biologischen Aufbereitung von Müll eine Nachrotte angeschlossen, um einen zusätzlichen Abbau an organischem Material und eine Trocknung des ausgelaugten Restes zu erreichen. Die Nachrotte kann problemlos auf offener Miete erfolgen. Der Anteil an biogenem Material ist auch nach der Perkolation genügend hoch, so daß die Rottetemperatur innerhalb von 4 bis 6 Tagen auf 70 ° C ansteigt. Innerhalb von 10 bis 16 Tagen erreicht der so behandelte Rückstand einen TS-Gehalt von bis zu 80 %. Da beim vorbeschriebenen Verfahren aufgrund der Biogasgewinnung und der Verstromung in einem Gasmotor Abwärme für eine Trocknung des Perkolationsrückstandes verfügbar wäre, kann auch ein platzsparendes Trocknungsverfahren als eine Nachrotte eingesetzt werden.

Die in Figur 3 dargestellten Anordnung wird gewählt, wenn ein kontinuierlicher Durchlaufbetrieb angestrebt wird. Bei großen Durchsatzleistungen kann die Anlage durch hinzufügen von weiteren Modulen (Reaktoren 1, Trockner 74) erweitert werden.

Die Fördereinrichtungen 36 und 84 und die Dosierer 34 (Materialumlenkung) sind derart ansteuerbar, daß die Reihenfolge der Befüllung, Entleerung oder Durch Mischung (Umlaufgut) der einzelnen Reaktoren, Trockner in beliebiger Reihenfolge verändert werden kann.

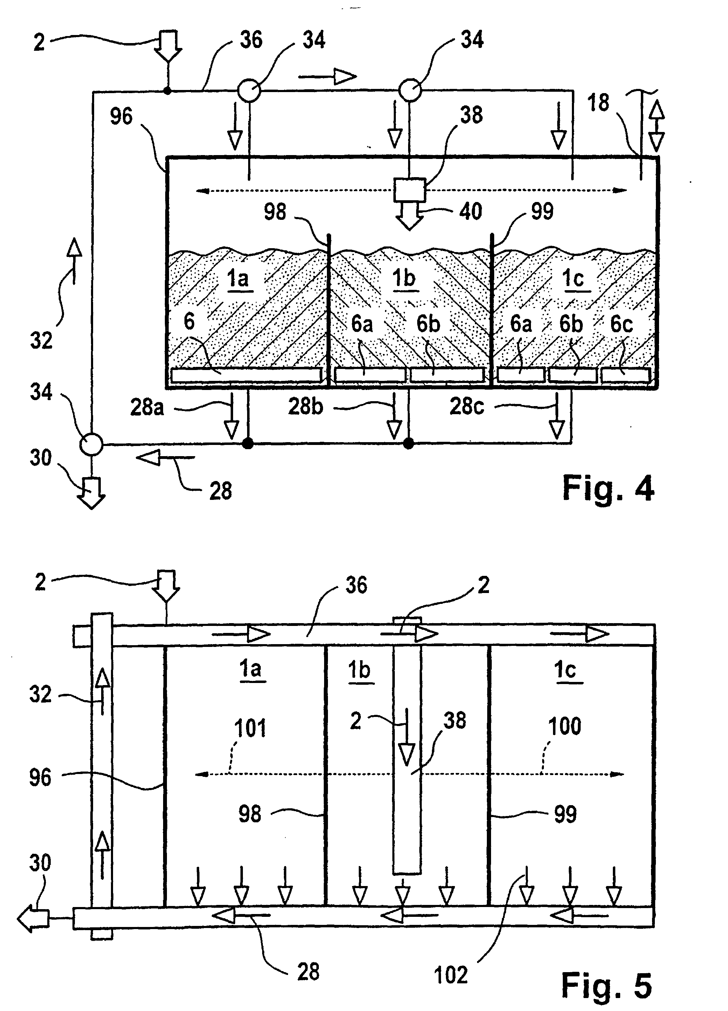

Figur 4 zeigt ein Ausführungsbeispiel, bei dem ein Behälter 96 durch zwei Trennwände in drei Kammern oder Reaktoren 1a, 1b, 1c aufgeteilt sind. Diese Kammern entsprechen den Einrichtungen gem. Figur 1 und gem. Figur 2 in denen die Hydrolyse und/oder die aerobe Trocknung durchführbar ist.

Dem dichten Behälter 96 ist eine gemeinsame Fördereinrichtung 36 zugeordnet, über die das aufzubereitende Stoffgemisch 2 dem Behälter 96 zugeführt wird. Von der gemeinsamen Fördereinrichtung 36 wird das Stoffgemisch über die Dosierer 34 zu einem Querförderer 38 geführt, der beim gezeigten Ausführungsbeispiel als Verteilerkran ausgeführt ist. Dieser hat einen Materialabwurf 40, der mittels des Verteilerkrans (Querförderers 38) über den gesamten Querschnittsbereich des Behälters 96 bewegbar ist. Dadurch ist gewährleistet, daß die Teilräume 1a, 1b, 1c des Behälters 96 geschichtet mit dem Stoffgemisch 2 beschickt werden können.

Der Austrag des aufbereitenden Stoffgemisches (Materialaustrag 28 oder Trockengut 76) erfolgt über eine Austragseinrichtung 6, die beispielsweise, diejenige in Figur 1 ausgeführt sein kann. Gemäß den in Figur 4 dargestellten Varianten können auch mehrere Austrageinrichtungen 6a, 6b, 6c nebeneinanderliegend im Bodenbereich des Behälters 96 ausgebildet werden. Beim gezeigten Ausführungsbeispiel ist der Behälter 96 als Mehrkammertrockner ausgeführt, so daß dieser mit Luftanschlüssen 14, 18 ausgeführt ist, wobei in Figur 4 lediglich der oben liegende Luftanschluß 18 gezeigt ist. Auch bei dieser Variante ist vorgesehen, daß die Luft im Gleichstrom oder im Gegenstrom zum Stoffgemisch geführt ist. Der Behälter 96 konnte selbstverständlich auch als Reaktor mit mehreren Teilkammern ausgeführt werden. Selbstverständlich können auch die in den Figuren 1 bis 3 dargestellten Reaktoren/Trockner mit mehreren nebeneinanderliegenden Austragseinrichtungen 6 ausgebildet werden.

Der Materialaustrag 28 kann über den Dosierer 34 wieder als Umlaufgut 32 zurück zur Fördereinrichtung 36 geführt werden oder aber als Produkt 30 abgeführt werden.

Figur 5 zeigt zur Verdeutlichung der Verteilung des Stoffgemisches 2 eine Draufsicht auf den Behälter 36 aus Figur 4. Demgemäß wird das Stoffgemisch 2 auf die Fördereinrichtung 36, beispielsweise ein Förderband aufgegeben und über dieses einem Verteilerkran 38 zugeführt, der in Pfeilrichtung 100, 101 oberhalb der Trennwände 98, 99 bewegbar ist. Der Verteilerkran 38 trägt einen bewegbaren oder mehrere stationäre Materialabwürfe 40, so daß die gesamte Breite (vertikalen Figur 5) der Teilkammern 1a, 1b, 1c überdeckbar ist.

Das aufbereitete Stoffgemisch wird in Pfeilrichtung 102 aus dem Behälter 96 ausgetragen und dieser Materialaustrag 28 über eine geeignete Fördereinrichtung entweder als Produkt 30 oder als Umlaufgut 32 abtransportiert. Letzteres wird über ein Förderband zurück zur Fördereinrichtung 36 transportiert und dann nochmals in einen der Teilräume 1a, 1b, 1c eingebracht.

Bei den vorbeschriebenen Ausführungsbeispielen wurde die Kaminbildung durch die über die Austragseinrichtung 6 ins Haufwerk eingeleiteten Kräfte verhindert, die eine wellenförmige Vertikalbewegung in der Schüttung verursachten und somit zu einer Neubildung der Schüttgutoberflächen und Zerstörung der Kanäle führte. In Abhängigkeit von der Güte des aufzubereitenden Stoffgemisches können die derart eingeleiteten Scherkräfte jedoch manchmal zu gering sein, um den erforderlichen mechanischen Aufschluß des Haufwerkes zu bewirken. Bei den vorbeschriebenen Reaktoren 1 wird dann der Anteil des Umlauf gutes 32 erhöht, so daß die zum Materialausschluß erforderlichen Scherkräfte über die Förderelemente zur Förderung des Umlaufguts 32 eingebracht werden.

Diese Variante ist hinsichtlich des energetischen und Materialaufwandes immer noch wesentlich günstiger als der eingangs genannte Stand der Technik, bei dem Rührwerke im Reaktorinneren zum Einbringen der Scherkräfte verwendet werden.

Der energetische und apparatetechnische Aufwand läßt sich weiter verringern, wenn der erfindungsgemäße Reaktor/Trockner in der in den Figuren 6 oder 7 beschriebenen Weise ausgeführt wird.

Bei den in den Figuren 1 und 2 beschriebenen Ausführungsbeispielen wurde die Prozessluft über einen oder mehrere Luftanschlüsse 14 in das Bodenteil des Reaktors 1 bzw. des Trockners eingeblasen wird und tritt dann durch den Siebboden 8 hindurch in die Schüttung (Haufwerk) ein. Im Unterschied dazu wird bei den in den Figuren 6 und 7 dargestellten Ausführungsbeispielen die Prozessluft über eine Vielzahl von über den Querschnitt des Reaktors 1 verteilten Lanzen 110 eingeblasen, deren Düsen 112 im unteren Bereich (Ansicht nach Figuren (6, 7) des Haufwerks 114 münden. Die Lanzen 110 durchsetzen dabei den Siebboden 8 und die Austragseinrichtung 6 - im vorliegenden Fall den Schubboden.

Die Prozessluft- oder Druckluft-Lanzen 110 sind über jeweils ein Steuerventil 116 mit einer Druckleitung 118 verbunden, die in einem Druckspeicher 120 mündet. Dieser ist an einen Kompressor 122 angeschlossen, über den Frischluft oder von der Abluftaufbereitung (Biofilter) zurückgeführte Luft 124 auf den Systemdruck, d.h. den Druck im Druckspeicher 120 bringbar ist. Die Steuerventile 116 sind an eine Prozeßsteuerung 126 angeschlossen und somit individuell auf- bzw. zusteuerbar.

Der Öffnungsquerschnitt der Steuerventile 116 kann dabei in Abhängigkeit von der Prozeßsteuerung 126 kontinuierlich verstellbar sein, so daß der Druck des Prozess-/Druckgases veränderbar ist.

Der Systemdruck im Druckspeicher 120 ist vorzugsweise auf einen Druck von mehr als 4 bar eingestellt. Beim vollständigen Öffnen eines Steuerventils 116 einer Lanze 110 tritt Druckluft 128 aus der Düsenmündung nach oben (Ansicht nach Figur 6, 7) aus und durchströmt das Haufwerk 114 in Vertikalrichtung mit dem Maximaldruck, wobei die Pfeile in den Figuren 6, 7 andeuten, daß sich die Druckluft 128 auch in Querrichtung abgelenkt wird. Das Haufwerk 114 wird im Durchströmungsbereich der Druckluft 128 partial aufgewirbelt oder fluidisiert, so daß die Oberflächen der Schüttung neu gebildet und Kanäle zerstört werden. D.h., durch die eingepreßte Druckluft wird eine partiell Wellenbewegung 130 im Haufwerk 114 erzeugt, die sich von der Düse 112 der jeweiligen Lanze 110 weg durch das Haufwerk 114 hindurch nach oben bewegt. Durch diese Wellenbewegung wird das Stoffgemisch relativ zueinander bewegt, so daß die Oberflächen der Partikel aufgerissen und somit die Stoff austauschfläche erhöht wird. Da die Druckluft nur impulsartig eingepreßt wird, sackt das Haufwerk 114 nach dem Schließen des Steuerventils 116 wieder zusammen, so daß wiederum Scherkräfte in das Haufwerk 114 eingeleitet werden, die zu einer nochmaligen Umbildung der Oberflächen und zur Zerstörung von Kanälen führen. Die aus dem Reaktor 1 austretende beladene Luft 123 wird einem Biofilter zugeführt.

Durch die eingepreßte Druckluft werden prinzipiell zwei Wirkungen erzielt. Zum einen werden in der vorbeschriebenen Weise Scherkräfte in das Haufwerk 114 eingeleitet, zum anderen wird auch die zur Hydrolyse und/oder Trocknung erforderliche Prozessluft zugeführt, so daß praktisch Scherkrafteintrag und Prozessluftzufuhr kombiniert sind. Modellrechnungen zeigten, daß sich durch die erfindungsgemäße Druckluftzufuhr der Energiebedarf gegenüber einem herkömmlichen Reaktor mit Rührwerk um bis zu mehr als 50 % verringern läßt.

Die Prozeßsteuerung 126 und die Steuerventile 116 sind derart ausgelegt, daß sich der Druck der Prozess-/Druckluft über der Zeit verändern läßt, so daß beispielsweise über einen vorbestimmten Zeitintervall lediglich Prozessluft mit geringem Druck (0,5 bar) zugeführt wird, die für die Trocknung oder Hydrolyse benötigt wird, jedoch zu keinem nennenswerten Scherkrafteintrag in das Haufwerk 114 führt. In Abhängigkeit von der Schütthöhe und der Qualität des aufzubereitenden Stoffgemisches wird dann intermittierend Durckluft mit vergleichsweise hohem Druck (> 4 bar) zugeführt, um die vorbeschriebene Scherkräfte einzubringen und Kanalbildungen zu vermeiden.

Die Ventile 116 der Vielzahl von Drucklanzen 110 des Reaktors 1 können auch auf einanderfolgend angesteuert werden, so daß das Haufwerk von einer "Expansionswelle" durchsetzt wird, die sich in der Darstellung gemäß den Figuren 6 und 7 parallel zur Zeichenebene oder senkrecht zur Zeichenebene bewegt.

Im übrigen entspricht das in Figur 6 dargestellte Ausführungsbeispiel den vorbeschriebenen Ausführungsbeispielen. D.h., das Stoffgemisch 2 wird über die Eintragseinrichtung 4 von oben geschichtet in den Reaktor 1 eingebracht und durchwandert diesen, wobei der Schichtaufbau durch die Druckluftzufuhr und die daraus resultierende Teilfluidisierung des Haufwerkes im wesentlichen unverändert bleibt. Das aufbereitete Stoffgemisch wird dann über die Austragseinrichtung 6, d.h. einen Schubboden abgeführt und den weiteren Aufbereitungsschritten zugeführt.

Bei dem in Figur 7 dargestellten Ausführungsbeispiel wird das Stoffgemisch 2 an der Figur 7 linken Stirnseite des Reaktors 1 zugeführt und an der gegenüberliegenden Seite des Reaktors 1 nach unten hin abgeführt. Demzufolge durchwandert das Stoffgemisch den Reaktionsraum 12 mit einem vertikal angeordneten Schichtaufbau, wie er mit den Bezugszeichen lₗ bis lₙ gekennzeichnet ist. D.h., das Stoffgemisch bewegt sich in Horizontalrichtung (1) durch den Reaktor, während es bei dem in Figur 6 dargestellten Ausführungsbeispiel in Vertikalrichtung durch den Reaktor bewegt wird.

Ansonsten entsprechen die in den Figuren 6 und 7 dargestellten Ausführungsbeispielen den vorherbeschriebenen Ausführungsbeispielen, so daß hinsichtlich der sonstigen Bauelemente auf die vorhergehenden Ausführungen verwiesen wird. Der Einfachheit halber wurden bei den Figuren 6 und 7 füreinander entsprechende Bauelemente die gleichen Bezugszeichen wie bei den Figuren 1 bis 5 verwendet.

Mit einfachen Worten gesagt, wird bei den in den Fi-guren 6 und 7 dargestellten erfindungsgemäßen Ausfuhrüngsbeispielen das beim Stand der Technik verwendete Rührwerk durch ein "Druckluftrührwerk" ersetzt, wobei der Druck der Druckluft derart gewählt wird, daß der Schichtaufbau im wesentlichen erhalten bleibt. Durch die individuelle Ansteuerbarkeit der über den Querschnitt des Reaktors 1 verteilten Steuerventile 116 kann das Haufwerk 114 gezielt mit Druckstössen beaufschlagt werden, so daß der Scherkrafteintrag prozeßabhängig, d.h. in Abhängigkeit von der Güte des aufzubereitenden Stoffgemisches und von der Verweildauer im Reaktor 1 mit Druckluft bzw. Prozessluft beaufschlagbar ist.

Offenbart ist ein Verfahren zur Aufbereitung eines Strukturanteile und Organik enthaltenen Stoffgemisches und eine Vorrichtung zur Durchführung dieses Verfahrens. Erfindungsgemäß wird das Stoffgemisch impulsartig oder periodisch mit Druckluft mit einem Druck von mehr als 2 bar beaufschlagt, so daß die Bildung von Strömungskanälen für eine Auswaschflüssigkeit oder Prozessluft in einem Haufwerk verhinderbar ist.

## Patentansprüche

1. Verfahren zur Aufbereitung eines Strukturanteile und Organik enthaltenen Stoffgemisches (2), das in einem Reaktor (1) als Haufwerk aufgenommen ist und mittels Durchströmung mit Prozessluft und/oder Zugabe einer Auswaschflüssigkeit einem aeroben Aufschluß oder einer aeroben Trocknung unterzogen wird, so daß die löslichen organischen Bestandteile abgeführt werden, **dadurch gekennzeichnet, daß** das Stoffgemisch (2) zum Einbringen von Scherkräften und zur Verhinderung von Kanalbildungen impulsartig oder periodisch mit etwa senkrecht und/oder parallel zur Stoffgemisch-Bewegungsrichtung gerichteter Druckluft mit einem Druck von mehr als 2 bar beaufschlagt wird.

2. Verfahren nach Patentanspruch 1, **dadurch gekennzeichnet, daß** die Druckluft bzw. die Prozessluft durch Düsen (112) im Kopfteil und/oder im Bodenteil des Reaktors (1) zugeführt wird.

3. Verfahren nach Patentanspruch 2, **dadurch gekennzeichnet, daß** die Prozessluft und die Druckluft durch die gleichen Düsen (112) zugeführt werden.

4. Verfahren nach einem der vorhergehenden Patentansprüche, **dadurch gekennzeichnet, daß** der Reaktor (1) kontinuierlich betrieben wird und das Stoffgemisch durch den Reaktor (1) etwa parallel oder im Querstrom zur Prozessluft geführt wird.

5. Verfahren nach einem der vorhergehenden Patentansprüche, **dadurch gekennzeichnet, daß** die Auswaschflüssigkeit durch einen Verteiler (44) im Kopfteil des Reaktors (1) zugeführt wird.

6. Verfahren nach einem der vorhergehenden Patentansprüche,
**dadurch gekennzeichnet, daß** die Druckluft mit einem Druck von mehr als 4 bar zugeführt wird.

7. Verfahren nach einem der vorhergehenden Patentansprüche, **dadurch gekennzeichnet, daß** das behandelte Stoffgemisch (2) über eine am Bodenteil des Reaktors (1) angeordnete Austragseinrichtung (6) abgezogen wird, über die die Kräfte ergänzend ins Haufwerk einbringbar sind.

8. Verfahren nach einem der vorhergehenden Patentansprüche, **dadurch gekennzeichnet, daß** sich an den aeroben Aufschluß eine aerobe Trocknung des Stoffgemisches anschließt.

9. Verfahren nach Patentanspruch 3, **dadurch gekennzeichnet, daß** das Stoffgemisch (2) aufeinanderfolgend mehrere Aufschlußund/oder Trocknungsschritte durchläuft.

10. Verfahren nach einem der vorhergehenden Patentansprüche, **dadurch gekennzeichnet, daß** sich an den Aufschluß- und/oder die aerobe Trocknung eine Kompaktierung des Stoffgemisches anschließt.

11. Vorrichtung, insbesondere zur Durchführung des Verfahrens gemäß einem der vorhergehenden Patentansprüche, mit einem Reaktor (1, 74, 96), dem eine Eintragseinrichtung (4) zum Einbringen eines Stoffgemisches zugeordnet ist, wobei im Bodenteil und/oder im Kopfteil des Reaktors (1, 74, 96) Einlässe (14, 18) zum Einbringen von Prozessluft und/oder im Kopfteil des Reaktors (1, 74, 96) ein Verteiler (20) für Aus-Waschflüssigkeit angeordnet sind, **gekennzeichnet durch** eine Druckluftanlage (6, 112), über die zum Aufschließen des Haufwerks (114) impulsförmig oder periodisch Druckluft mit mehr als 2 bar zuführbar ist, und **durch** eine Austragseinrichtung (6) im Bodenteil des Reaktors (1, 76, 96).

12. Vorrichtung nach Patentanspruch 11, **dadurch gekennzeichnet, daß** die Druckluftanlage im Bodenteil des Reaktors (1) mündende Düsen (112) hat, die an einen von einem Kompressor (122) gespeisten Druckspeicher (120) angeschlossen sind.

13. Vorrichtung nach Patentanspruch 11 oder 12, **dadurch gekennzeichnet, daß** der Druckluftanlage eine Steuerung (126) zugeordnet ist, über die der Druck der Druckluft oder Prozessluft variierbar ist.

14. Vorrichtung nach einem der Patentansprüche 11 bis 13, **dadurch gekennzeichnet, daß** über die Druckluftanlage Druckluft *und* Prozessluft gemeinsam zuführbar sind.

15. Vorrichtung nach einem der Patentansprüche 11 bis 14, **gekennzeichnet durch** eine Gasreinigungsanlage zur Reinigung und Rückführung der Prozess- und/oder Druckluft.

16. Vorrichtung nach einem der Patentansprüche 11 bis 15, **dadurch gekennzeichnet, daß** die Erregungseinrichtung zumindest teilweise durch eine Austragseinrichtung (6) am Bodenteil des Reaktors (1, 74, 96) gebildet ist.

17. Vorrichtung nach einem der Patentansprüche 11 bis 16, **dadurch gekennzeichnet, daß** mehreren Reaktoren (1, 74, 96) in Reihe geschaltet sind, wobei mehrere Reaktoren (1, 74, 96) eine gemeinsame Eintrageinrichtung (4) zugeordnet ist, über die das aufzubereitende Stoffgemisch (2) zuführbar ist.

18. Vorrichtung nach einem der Patentansprüche 11 bis 17, **gekennzeichnet durch** eine Kompaktierungseinrichtung (90) zur Kompaktierung, Entwässerung und Formgebung des aufbereitenden Stoffgemisches.

## Claims

1. A process for treating a mixture of substances (2) containing structured constituents and organic matter which is received in the form of bulk material and subjected to aerobic decomposition or aerobic drying in a reactor (1) by means of a flow of process air therethrough and/or addition of a leaching fluid so that the soluble organic constituents are discharged, **characterized in that** said mixture of substances (2) is subjected to pulse-type or periodical application of pressurized air having a pressure of more than 2 bar and directed approximately perpendicular to and/or in parallel with the direction of displacement of said mixture of substances so as to introduce shear forces and prevent channel formation.

2. The process according to claim 1, **characterized in that** the pressurized air or process air, respectively, is supplied through nozzles (112) in the head portion and/or in the bottom portion of said reactor (1).

3. The process according to claim 2, **characterized in that** the process air and the pressurized air are supplied through said same nozzles (112).

4. The process according to any one of the preceding claims, **characterized in that** said reactor (1) is operated continuously, and the mixture of substances is guided through said reactor (1) approximately in parallel with or transversally to the process air.

5. The process according to any one of the preceding claims, **characterized in that** the leaching fluid is supplied through a distributor (44) in the head portion of said reactor (1).

6. The process according to any one of the preceding claims, **characterized in that** the pressurized air is supplied at a pressure of more than 4 bar.

7. The process according to any one of the preceding claims, **characterized in that** said treated mixture of substances (2) is withdrawn through a discharge means (6) arranged at the bottom portion of said reactor (1), through which the forces may supplementarily be introduced into the bulk material.

8. The process according to any one of the preceding claims, **characterized in that** said aerobic decomposition is followed by aerobic drying of said mixture of substances.

9. The process according to claim 3, **characterized in that** said mixture of substances (2) successively passes through a plurality of decomposition and/or drying steps.

10. The process according to any one of the preceding claims, **characterized in that** said decomposition and/or aerobic drying is followed by compacting of the mixture of substances.

11. A device, in particular for implementing the process in accordance with claim 1, including a reactor (1, 74, 96) having associated a charging means (4) for introduction of a mixture of substances, wherein inlets (14, 18) for introducing process air are arranged in the bottom portion and/or in the head portion of said reactor (1, 74, 96), and/or a distributor (20) for leaching fluid is arranged in the head portion of said reactor (1, 74, 96), **characterized by** a pressurized air system (6, 112) whereby pressurized air of more than 2 bar may be supplied in the form of pulses or periodically for decomposition of said bulk material (114), and by discharge means (6) in the bottom portion of said reactor (1, 74, 96).

12. The device according to claim 11, **characterized in that** said pressurized air system includes nozzles (112) opening in the bottom portion of said reactor (1) and connected to a pressure accumulator (120) fed by a compressor (122).

13. The device according to claim 11 or 12, **characterized in that** said pressurized air system is associated with a control means (126) through which the pressure of pressurized air or process air may be varied.

14. The device according to any one of claims 11 to 13, **characterized in that** pressurized air and process air may be supplied jointly through said pressurized air system.

15. The device according to any one of claims 11 to 14, **characterized by** a gas purification system for purification and recirculation of the process air and/or pressurized air.

16. The device according to any one of claims 11 to 15, **characterized in that** said excitation means is at least partly constituted by a discharge means (6) at the bottom portion of said reactor (1, 74, 96).

17. The device according to any one of claims 11 to 16, **characterized in that** a plurality of reactors (1, 74, 96) are arranged in series, with several reactors (1, 74, 96) having associated a common charging means (4) whereby said mixture of substances (2) to be treated may be supplied.

18. The device according to any one of claims 11 to 17, **characterized by** a compacting means (90) for compacting, dewatering and shaping said mixture of substances to be treated.

## Revendications

1. Procédé de traitement d'un mélange de substances (2) contenant des éléments structurels et organiques, qui est introduit dans un réacteur (1) sous la forme de déblais et soumis à une désagrégation aérobic ou à un séchage aérobic à l'aide d'un flux d'air de process et/ou d'une addition d'un liquide de lavage, de telle sorte que les composants organiques solubles sont éliminés, **caractérisé en ce que**, sur le mélange de substances (2), de l'air comprimé d'une pression supérieure à 2 bars est appliqué par impulsions ou périodiquement, qui est orienté sensiblement verticalement et/ou parallèlement au sens de déplacement du mélange de substances, pour introduire des forces de cisaillement et pour éviter la formation de canaux.

2. Procédé selon la revendication 1,
**caractérisé en ce que** l'air comprimé ou l'air de process est introduit par des buses (112) prévues dans la partie de tête et/ou dans la partie de fond du réacteur (1).

3. Procédé selon la revendication 2,
**caractérisé en ce que** l'air de process ou l'air comprimé est introduit par les mêmes buses (112).

4. Procédé selon l'une quelconque des revendications précédentes, **caractérisé en ce que** le réacteur (1) fonctionne en continu, et **en ce que** le mélange de substances est conduit à travers le réacteur sensiblement parallèlement ou transversalement à l'air de process.

5. Procédé selon l'une quelconque des revendications précédentes, **caractérisé en ce que** le liquide de lavage est introduit par un répartiteur (44) prévu dans la partie de tête du réacteur (1).

6. Procédé selon l'une quelconque des revendications précédentes, **caractérisé en ce que** l'air comprimé est introduit avec une pression supérieure à 4 bars.

7. Procédé selon l'une quelconque des revendications précédentes, **caractérisé en ce que** le mélange de substances (2) traité est extrait par un dispositif d'extraction (6) disposé sur la partie de fond du réacteur (1), dispositif permettant également d'introduire des forces dans les déblais.

8. Procédé selon l'une quelconque des revendications précédentes, **caractérisé en ce que** la désagrégation aérobic est suivie d'un séchage aérobic du mélange de substances.

9. Procédé selon la revendication 3,
**caractérisé en ce que** le mélange de substances (2) est successivement soumis à plusieurs étapes de désagrégation et/ou de séchage.

10. Procédé selon l'une quelconque des revendications précédentes, **caractérisé en ce que** la désagrégation et/ou le séchage aérobics sont suivis d'un compactage du mélange de substances.

11. Dispositif, notamment pour la mise en oeuvre du procédé selon l'une quelconque des revendications précédentes, comprenant un réacteur (1, 74, 96) auquel est associée une unité d'introduction (4) pour l'introduction d'un mélange de substances, dans la partie de fond et/ou dans la partie de tête du réacteur (1, 74, 96) des entrées (14, 18) pour l'introduction d'air de process, et /ou dans la partie de tête du réacteur (1, 74, 96) un répartiteur (20) pour un liquide de lavage étant disposés, **caractérisé par** une installation d'air comprimé (6, 112) permettant d'introduire sous la forme d'impulsions ou périodiquement de l'air comprimé d'une pression supérieure à 2 bars pour désagréger les déblais, et par une unité d'extraction (6) prévue dans la partie de fond du réacteur (1, 74, 96).

12. Dispositif selon la revendication 11,
**caractérisé en ce que** l'installation d'air comprimé présente des buses (112) débouchant dans la partie de fond du réacteur, qui sont raccordées à un accumulateur de pression (120) alimenté par un compresseur (122).

13. Dispositif selon la revendication 11 ou 12, **caractérisé en ce qu'**à l'installation d'air comprimé une commande (126) est associée permettant de varier la pression de l'air comprimé ou de l'air de process.

14. Dispositif selon l'une quelconque des revendications 11 à 13, **caractérisé en que** l'installation d'air comprimé permet d'introduire conjointement de l'air comprimé et de l'air de process.

15. Dispositif selon l'une quelconque des revendications 11 à 14, **caractérisé par** une installation de nettoyage à gaz pour nettoyer et reconduire l'air de process et/ou l'air comprimé.

16. Dispositif selon l'une quelconque des revendications 11 à 15, **caractérisé en ce que** l'unité d'excitation est au moins partiellement constituée par une unité d'extraction (6) prévue sur la partie de fond du réacteur (1, 74, 96).

17. Dispositif selon l'une quelconque des revendications 11 à 16, **caractérisé en ce que** plusieurs réacteurs (1, 74, 96) sont montés en série, une unité d'introduction (4) commune étant associée à plusieurs réacteurs (1, 74, 96), par laquelle le mélange de substances (2) à traiter peut être introduit.

18. Dispositif selon l'une quelconque des revendications 11 à 17, **caractérisé par** une unité de compactage (90) pour compacter, drainer et mettre en forme le mélange de substances traité.
